# FASCICULE DE BREVET EUROPEEN

(11) **EP 1 856 081 B1**
(45) Date de publication et mention de la délivrance du brevet: **15.12.2010**
(21) Numéro de dépôt: 06709353.4
(22) Date de dépôt: 21.02.2006
(51) Int. Cl.: C07D 305/14

(54) **PROCEDE DE PREPARATION DU PACLITAXEL**
VERFAHREN ZUR HERSTELLUNG VON PACLITAXEL
METHOD FOR PREPARING PACLITAXEL

(30) Priorité: 23.02.2005 FR 0501838
(43) Date de publication de la demande: 21.11.2007
(73) Titulaire: Seripharm, 72650 Le Mans (FR)
(72) Inventeur: LEZE, Antoine, Paul, Gaston, F-72650 La Milesse (FR)
(74) Mandataire: Hirsch & Associés
(86) Numéro de dépôt international: PCT/FR2006/000387
(87) Numéro de publication internationale: WO 2006/090057

(56) Documents cités:
- WO-A-00/69840
- WO-A-94/07878
- WO-A-2006/004708
- US-A1- 2002 161 238
- US-B1- 6 222 053
- US-B1- 6 365 750
- KINGSTON D ET AL: "SYNTHESIS OF TAXOL FROM BACCATIN III" TETRAHEDRON LETTERS, ELSEVIER, AMSTERDAM, NL, vol. 35, no. 26, 1994, pages 4483-4484, XP002099799 ISSN: 0040-4039
- HOLTON R A ET AL: "Selective Protection of the C(7) and C(10) Hydroxyl Groups in 10-Deacetyl Baccatin III" 7 mai 1998 (1998-05-07), TETRAHEDRON LETTERS, ELSEVIER, AMSTERDAM, NL, PAGE(S) 2883-2886 , XP004115707 ISSN: 0040-4039 le document en entier

## Description

La présente invention concerne un procédé de préparation du paclitaxel à partir de la 10-déacétylbaccatine (10-DAB).

Dans la demande de brevet WO 94/07878 a été décrit le procédé qui consiste dans la préparation de dérivés du taxane, notamment du paclitaxel à partir de la 10-DAB protégée ou de la baccatine III en passant intermédiairement par un dérivé de baccatine III à chaîne cyclique oxazolidine, puis ouverture de cette chaîne. Cependant les différentes étapes, notamment les étapes préalables de préparation de 10-DAB protégée ou de la baccatine III ne sont pas spécifiquement décrites et n'avaient jamais été optimisées en vue d'une mise en oeuvre industrielle performante, ce qui ne permettait pas d'atteindre des résultats aussi satisfaisants que souhaités pour une application industrielle à plus grande échelle.

Dans la demande de brevet WO 2006/004708 a été décrit le procédé de protection d'un taxane de formule : 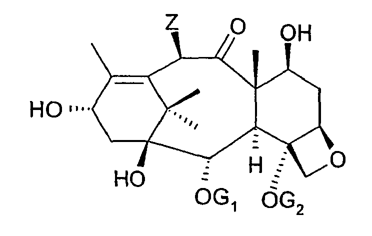 dans laquelle, Z est -OH ou un -OH protégé, G₁ and G₂ sont identiques ou différents et représentent independamment un groupement protecteur d'hydroxy, le procédé comprenant : la protection des hydroxy libres en position C-7 et/ou en position C-10 du taxane; et la condensation d'une chaine latérale sur l'hydroxy en position C-13 du taxane pour préparer un taxane intermediaire C-13 protégé, et dans lequel les étapes de protection et de condensation sont caractérisées en ce que dans une réaction « one-pot » le taxane est combiné avec une base, un agent protecteur d'hydroxy convenable et un précurseur de chaîne latérale et dans lequel le précurseur de chaîne latérale est un β-lactame, une oxazolidine ou une oxazoline.

Selon l'invention, il a maintenant été trouvé que le paclitaxel peut être avantageusement préparé à partir de la 10-déacétylbaccatine selon une réaction « one-pot » impliquant les 3 étapes successives suivantes :
a) protection du radical hydroxy en position -7 de la 10-DAB par un radical silylé, puis
b) acétylation du radical hydroxy en position -10,
c) éventuellement cristallisation du dérivé de baccatine III obtenu,
   suivies de la condensation de l'acide (4S,SR)-3-N-benzoyl-2RS-méthoxy-4-phényl-1,3-oxazolidine-5-carboxylique de formule : 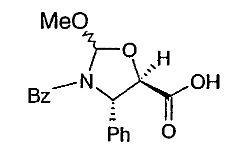 par estérification en position -13 du dérivé 10-acétylé de baccatine III obtenu précédemment, de formule générale : 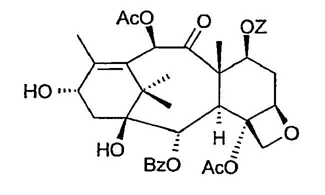 dans laquelle Z est un radical protecteur silylé, pour obtenir un dérivé de formule générale : 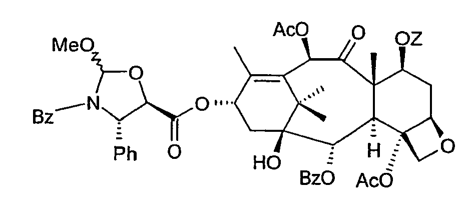 dans laquelle Z est un radical protecteur silylé, puis ouverture de l'oxazolidine de la chaîne latérale cyclique et libération simultanée du radical hydroxy en position -7, de son radical protecteur et purification éventuelle du paclitaxel obtenu.
   Le radical protecteur Z est choisi parmi des radicaux protecteurs silylés habituellement utilisés dans la chimie des taxanes et dont la mise en place et l'élimination n'altère pas le reste de la molécule. Notamment, le radical protecteur d'hydroxy en position -7, peut être choisi parmi les groupements trialkylsilyle, dialkylarylsilyle, alkyldiarylsilyle ou triarylsilyle dans lesquelles les radicaux alkyles contiennent 1 à 4 C en chaîne droite ou ramifiée et aryle est de préférence phényle, par exemple des radicaux triéthylsilyle ou triméthylsilyle, de préférence triéthylsilyle.

La protection du radical hydroxy en position -7 de la 10-DAB par un radical silylé s'effectue avantageusement par action du chlorure de triéthylsilyle en présence de pyridine (pyridine anhydre) selon la méthode décrite dans le brevet européen EP336840, ou en présence de diméthylamino-4 pyridine dans un solvant comme le dichlorométhane selon la méthode décrite dans la demande internationale WO 94/14787. La réaction s'effectue à une température comprise entre 0 et 10°C, de préférence à une température comprise entre 5 et 10°C.

L'acétylation b) de la 10-DAB silylée est mise en oeuvre par action du chlorure d'acétyle ou de l'anhydride acétique dans les conditions décrites dans le brevet européen EP336840. De préférence on opère par addition de l'anhydride acétique dans la pyridine, à une température comprise entre 0 et 25°C, de préférence à une température voisine de 20°C.

Lorsque l'on veut purifier le dérivé acétylé obtenu, la cristallisation du dérivé de baccatine III protégé en position -7 est avantageusement mise en oeuvre dans un mélange méthanol / eau 50/50 en volumes. De préférence on utilise la triéthylsilyloxy-7 baccatine III et l'on opère à une température comprise entre 0 et 30°C suivie du maintien à environ 10°C.

Il est entendu que les 3 étapes de protection du radical hydroxy en position-7, d'acétylation en position -10 et éventuellement de cristallisation du dérivé de baccatine III protégé en position -7, s'opèrent sans isolement des produits intermédiaires formés.

L'estérification du dérivé 10-acétylé de baccatine III, de formule générale : 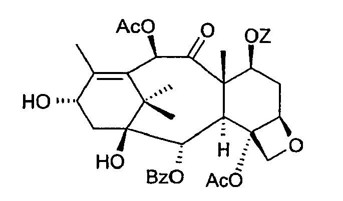 dans laquelle Z est un radical protecteur silylé, par l'acide (4S,5R)-3-N-benzoyl-2RS-méthoxy-4-phényl-1,3-oxazolidine-5-carboxylique s'effectue en présence d'un agent de condensation comme par exemple un carbodiimide (dicyclohexylcarbodiimide par exemple) et d'une base azotée comme une aminopyridine (4-diméthylaminopyridine, 4-pyrrolidinopyridine). La réaction est mise en oeuvre dans un solvant organique comme un éther, un ester (acétate d'éthyle par exemple), un nitrile ou un hydrocarbure aliphatique ou aromatique à une température comprise entre -10 et 90°C, de préférence à une température comprise entre 10 et 30°C et plus particulièrement entre 20 et 25°C. Avantageusement, l'ester ainsi obtenu est cristallisé en présence de méthanol.

L'ouverture de l'oxazolidine de la chaîne latérale cyclique et libération simultanée du radical hydroxy en position -7 s'effectuent dans un solvant organique comme un ester (acétate d'éthyle par exemple) ou un alcool (éthanol, méthanol par exemple) ou dans un mélange de ces solvants, par exemple le mélange acétate d'éthyle / éthanol, par addition d'un acide comme par exemple un acide halogéné, acide chlorhydrique par exemple, à une température comprise entre 35 et 55°C, de préférence à une température comprise entre 40 et 50°C, puis par addition d'eau. De préférence on opère sous atmosphère inerte. Le paclitaxel brut ainsi obtenu est purifié par chromatographie et cristallisation.

Des conditions opératoires sont également décrites dans la demande internationale WO 94/07878.

L'exemple suivant illustre la présente invention.

### Exemple 1 :

### 7-O-Triéthylsilylbaccatine III

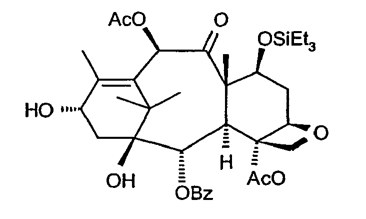

A une solution agitée à 5°C sous atmosphère inerte de 1 g (1,84 mmol) de 10-désacétylbaccatine III dans 2 ml de pyridine anhydre, on additionne 0,42 g (2,79 mmol) de chlorotriéthylsilane en 30 min. Après 24 heures de réaction à 5°C, on additionne 0,37 g (3,62 mmol) d'anhydride acétique en 30 minutes puis le mélange réactionnel est ramené à température ambiante. Après 22 h de réaction, on verse 5 mL d'un mélange méthanol/eau (50/50) en 30 minutes. La suspension est maintenue agitée pendant 30 minutes puis elle est filtrée sur verre fritté.

Après séchage, on obtient ainsi 1,09 g du composé cité ci-dessus en titre, sous la forme d'un solide blanc (Rdt = 85%).

Le composé obtenu présente les caractéristiques suivantes :
RMN ¹H 400 MHz (CDCl₃) (δ ppm) : 8,11 (2H, d, J = 7,1 Hz) ; 7,6 (1H, t, J = 7,4 Hz) ; 7,48 (2H, t, J = 7,7 Hz) ; 6,46 (1H, s) ; 5,63 (1H, d, J = 7 Hz) ; 4,96 (1H, d, J = 8,1 Hz) ; 4,83 (1H, m) ; 4,49 (1H, dd, J = 10,4 et 6,7 Hz) ; 4,31 et 4,15 (2H , 2d, J = 8,3 Hz) ; 3,38 (1H, d, J = 7 Hz) ; 2,53 (1H, m) ; 2,29 (3H, s) ; 2,27 (2H, m) ; 2,19 (3H, d, J = 0,8 Hz) ; 2,18 (3H, s) ; 2,12 (1H, d) ; 1,88 (1H, m) ; 1,68 (3H, s) ; 1,65 (1H, s) ; 1,2 (3H, s) ; 1,04 (3H, s) ; 0,92 (9H, t) ; 0,59 (6H, m).

### Exemple 2 :

### 13-O-[[(4S,SR)-3-N-benzoyl-4-phényloxazolidin-2RS-méthoxy-5-yl]-carbonyl]-7-O-triéthylsilylbaccatine III

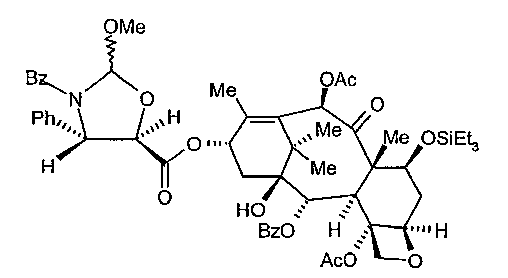

A une solution agitée sous atmosphère inerte à température ambiante de 0,58 g (1,77 mmol) d'acide (4S,5R)-3-*N*-benzoyl-4-phényloxazolidin-2RS-méthoxy-5-carboxylique dans 6,5 mL d'acétate d'éthyle, on ajoute 1 g (1,43 mmol) de 7-*O*-triéthylsilylbaccatine III. On ajoute une solution de 0,73 g (3,54 mmol) de dicyclohexylcarbodiimide dans 1 mL d'acétate d'éthyle et 0,02 g (0,16 mmol) de 4-diméthylaminopyridine. Après 1 heure de réaction, les insolubles sont éliminés par filtration et la phase organique est concentrée sous pression réduite.

On obtient ainsi 1,36 g du composé cité ci-dessus en titre sous la forme d'un résidu jaunâtre (Rdt = 95%).

Le composé obtenu présente les caractéristiques suivantes :
RMN ¹H 400 MHz (CDCl₃) (δ ppm) : 8,13 (2H, d, J = 7,3 Hz) ; 7,75 (2H, d, J = 7,3 Hz) ; 7,61 (1H, t, J = 7,4 Hz) ; 7,50 (5H, m) ; 7,41 (5H, m) ; 7,05 (1H, d, J = 9,0) ; 6,43 (1H, s) ; 6,19 (1H, t, J = 8,6 Hz) ; 5,80 (1H, dd, J = 8,9 et 2,4 Hz) ; 5,69 (1H, d, J = 7 Hz) ; 4,92 (1H, d, J = 8,1 Hz) ; 4,80 (1H, d, J = 2,4 Hz) ; 4,44 (1H, dd, J = 10,4 et 6,7 Hz) ; 4,30 et 4,19 (2H , 2d, J = 8,4 Hz) ; 3,80 (1H, d, J = 7 Hz) ; 3,63 (1H, s) ; 2,52 (1H, m) ; 2,38 (3H, s) ; 2,31 (2H, m) ; 2,18 (3H, s) ; 1,91 (3H, d, J = 0,6 Hz) ; 1,88 (1H, m) ; 1,70 (3H, s) ; 1,23 (3H, s) ; 1,18 (3H, s) ; 0,97 (6H, t, J = 8,0 Hz) ; 0,92 (3H, t, J = 8,0 Hz) ; 0,59 (6H, m).

### Exemple 3 :

### Paclitaxel

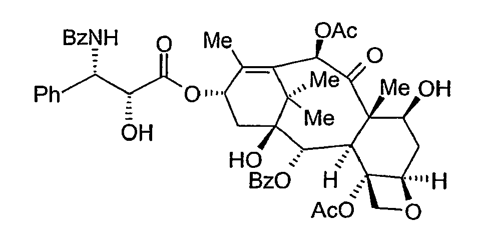

A une solution agitée chauffée à 45°C sous atmosphère inerte de 1 g (1 mmol) de 13-*O-*[[(4S,SR)-3-*N*-benzoyl-4-phényloxazolidin-2RS-méthoxy-5-yl]-carbonyl]-7-*O-*triéthylsilylbaccatine III dans mélange de 8 mL d'acétate d'éthyle et de 4 mL d'éthanol, on ajoute 2 mL (3 mmol) d'une solution aqueuse d'HCl 1,5N. Après 1 heure de réaction, on verse 2 mL d'eau et le mélange réactionnel est agité 3 heures supplémentaires à 45°C. Après addition de 16 mL d'acétate d'éthyle, la phase organique est lavée avec 11 mL d'une solution aqueuse saturée d'hydrogénocarbonate de sodium puis avec 11 mL d'une solution aqueuse saturée de chlorure de sodium et concentrée sous pression réduite.

Après chromatographie du produit brut sur gel de silice (10 µm) (éluant : cyclohexane-acétate d'éthyle, 5/5) et cristallisation dans un mélange d'éthanol et d'eau (25/75), on isole ainsi 0,65 g de paclitaxel à l'état cristallisé (Rdt = 80%).

## Revendications

1. Un procédé de préparation du paclitaxel **caractérisé en ce que** l'on opère à partir de la 10-déacétylbaccatine selon une réaction « one-pot » impliquant les 3 étapes successives suivantes :
a) protection du radical hydroxy en position -7 de la 10-DAB par un radical silylé, puis
b) acétylation du radical hydroxy en position -10,
c) éventuellement cristallisation du dérivé de baccatine III obtenu,
suivies de la condensation de l'acide (4S,SR)-3-N-benzoyl-2RS-méthoxy-4-phényl-1,3-oxazolidine-5-carboxylique de formule : 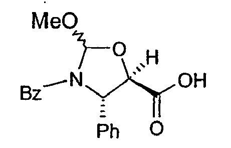 par estérification en position -13 du dérivé 10-acétylé de baccatine III obtenu précédemment, de formule générale : 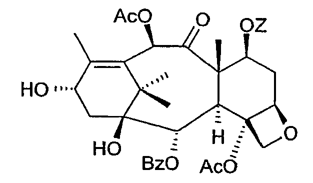 dans laquelle Z est un radical protecteur silylé, pour obtenir un dérivé de formule générale : 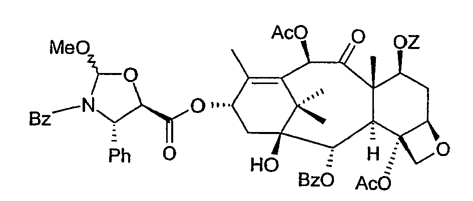 dans laquelle Z est un radical protecteur silylé, puis ouverture de l'oxazolidine de la chaîne latérale cyclique et libération simultanée du radical hydroxy en position -7, de son radical protecteur et
purification éventuelle du paclitaxel obtenu.

2. Un procédé selon la revendication 1, **caractérisé en ce que** le radical protecteur silylé est choisi parmi les groupements trialkylsilyle, dialkylarylsilyle, alkyldiarylsilyle ou triarylsilyle dans lesquelles les radicaux alkyles contiennent 1 à 4 C en chaîne droite ou ramifiée et aryle représente phényle.

3. Un procédé selon la revendication 2, **caractérisé en ce que** le radical protecteur silylé est choisi parmi les radicaux triéthylsilyle ou triméthylsilyle.

4. Un procédé selon l'une des revendications 1 à 3, **caractérisé en ce que** le dérivé de baccatine III protégé en position-7 est cristallisé dans un mélange méthanol / eau.

5. Un procédé selon la revendication 4, **caractérisé en ce que** le dérivé de baccatine III protégé en position-7 est la triéthylsilyloxy-7 baccatine III.

## Claims

1. A process for preparing paclitaxel, **characterized in that** the process is carried out from 10-deacetyl baccatin according to a "one-pot" reaction involving the following 3 successive steps:
a) protection of the hydroxyl radical in the 7-position of the 10-DAB, with a silylated radical, then
b) acetylation of the hydroxyl radical in the 10-position,
c) optionally, crystallization of the baccatin III derivative obtained, followed by condensation of (4S,5R)-3-N-benzoyl-2RS-methoxy-4-phenyl-1,3-oxazolidine-5-carboxylic acid of formula: 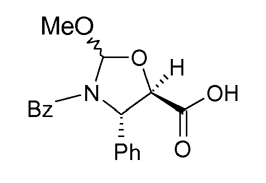 by esterification in the 13-position of the 10-acetylated derivative of baccatin III obtained above, of general formula: 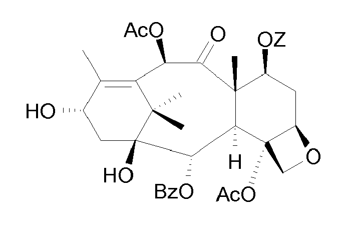 in which Z is a silylated protective radical, so as to obtain a derivative of general formula: 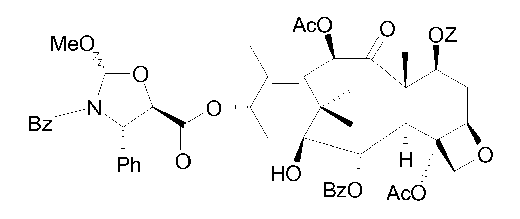 in which Z is a silylated protective radical, and then opening of the oxazolidine of the cyclic side chain and simultaneous deprotection of the hydroxyl radical in the 7-position, from its protective radical, and
optional purification of the paclitaxel obtained.

2. The process as claimed in claim 1, **characterized in that** the silylated protective group is chosen from trialkylsilyl, dialkylarylsilyl, alkyldiarylsilyl or triarylsilyl groups in which the alkyl radicals contain 1 to 4 carbons in a straight or branched chain, and aryl represents phenyl.

3. The process as claimed in claim 2, **characterized in that** the silylated protective radical is chosen from triethylsilyl or trimethylsilyl radicals.

4. The process as claimed in one of claims 1 to 3, **characterized in that** the baccatin III derivative protected in the 7-position is crystallized from a methanol/water mixture.

5. The process as claimed in claim 4, **characterized in that** the baccatin III derivative protected in the 7-position is 7-triethylsilyloxy-baccatin III.

## Patentansprüche

1. Verfahren zur Herstellung von Paclitaxel, **dadurch gekennzeichnet, dass** es ausgehend von 10-Deacetyl-baccatin gemäß einer "Eintopfreaktion" betrieben wird, an welcher die folgenden nacheinander durchgeführten Schritte beteiligt sind:
a) Schützen der Hydroxygruppe an Position -7 des 10-DAB durch eine silylartigen Gruppe, woraufhin
b) eine Acetylierung der Hydroxygruppe an Position -10 erfolgt,
c) das erhaltene Baccatin-III-Derivat möglicherweise eine Kristallisation erfährt,
woraufhin die (4S,5R)-3-N-Benzoyl-2RS-metboxy-4-phenyl-1,3-oxazoliden-5 - carbonsäure der Formel: 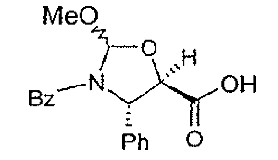 eine Kondensationsreaktion erfährt, indem sie an Position -13 des vorstehend erhaltenen 10-Acetyl-derivats des Baccatins-III mit der folgenden allgemeinen Formel: 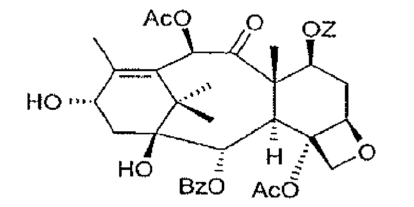 in welcher Z eine silylartige Schutzgruppe ist, eine Esterbindung eingeht, um ein Derivat der folgenden allgemeinen Formel zu erhalten: 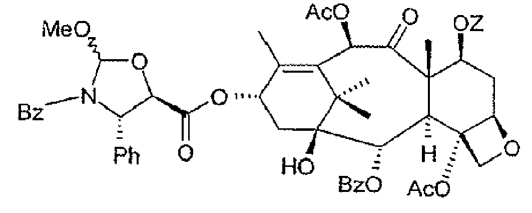 in welcher Z eine silylartige Schutzgruppe ist, woraufhin das Oxazolidin der zyklischen Seitenkette geöffnet und gleichzeitig die Schutzgruppe von der Hydroxygruppe an Position -7 entfernt wird und
das erhaltene Paclitaxel möglicherweise aufgereinigt wird.

2. Verfahren nach Anspruch 1, **dadurch gekennzeichnet, dass** die silylartige Schutzgruppe aus den Gruppen Trialkylsilyl, Dialkylarylsilyl, Alkyldiarylsilyl oder Triarylsilyl gewählt ist, in welchen die Alkylgruppen 1 bis 4 C in geradkettiger oder verzweigter Anordnung enthalten und Aryl für Phenyl steht.

3. Verfahren nach Anspruch 2, **dadurch gekennzeichnet, dass** die silylartige Schutzgruppe aus den Gruppen Triethylsilyl oder Trimethylsilyl gewählt ist.

4. Verfahren nach einem der Ansprüche 1 bis 3, **dadurch gekennzeichnet, dass** das Baccatin-III-Derivat, das an Position -7 geschützt ist, in einer Mischung aus Methanol / Wasser kristallisiert wird.

5. Verfahren nach Anspruch 4, **dadurch gekennzeichnet, dass** es sich bei dem Baccatin-III-Derivat, das an Position -7 geschützt ist, um 7-Triethylsilyloxy-Baccatin-III handelt.
